(19) Europäisches Patentamt
European Patent Office
Office européen des brevets

(11) **EP 3 484 346 B1**

(12) **EUROPEAN PATENT SPECIFICATION**

(45) Date of publication and mention
of the grant of the patent:
**21.12.2022 Bulletin 2022/51**

(21) Application number: **17746009.4**

(22) Date of filing: **14.07.2017**

(51) International Patent Classification (IPC):
**A61B 5/00** *(2006.01)*    **A61B 5/02** *(2006.01)*
**A61B 5/021** *(2006.01)*    **A61B 5/0215** *(2006.01)*

(52) Cooperative Patent Classification (CPC):
**A61B 5/6851; A61B 5/02007; A61B 5/02108;**
**A61B 5/02158; A61B 5/7221; A61B 5/7246**

(86) International application number:
**PCT/EP2017/067903**

(87) International publication number:
**WO 2018/011411 (18.01.2018 Gazette 2018/03)**

(54) **APPARATUS, SYSTEM AND METHOD FOR FEEDBACK ON QUALITY OF PROPERTY MEASUREMENT IN A VESSEL**

VORRICHTUNG, SYSTEM UND VERFAHREN ZUR RÜCKMELDUNG ÜBER DIE QUALITÄT VON EIGENSCHAFTSMESSUNGEN IN EINEM GEFÄSS

APPAREIL, SYSTÈME ET PROCÉDÉ DE RETOUR D'INFORMATIONS SUR LA QUALITÉ DE MESURE DE PROPRIÉTÉ DANS UN VAISSEAU

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **14.07.2016 EP 16179420**

(43) Date of publication of application:
**22.05.2019 Bulletin 2019/21**

(73) Proprietor: **Koninklijke Philips N.V.**
**5656 AG Eindhoven (NL)**

(72) Inventor: **VAN DEN BRINK, Hendrikus, Bernardus**
**5656 AE Eindhoven (NL)**

(74) Representative: **Philips Intellectual Property & Standards**
**High Tech Campus 52**
**5656 AG Eindhoven (NL)**

(56) References cited:
**WO-A1-2016/030491    US-A1- 2010 234 698**
**US-A1- 2014 276 036    US-A1- 2016 073 972**
**US-A1- 2016 106 373**

- **PERREY C ET AL: "Evaluation of intracoronary pressure and blood flow velocity for the assessment of coronary stenoses severity", COMPUTERS IN CARDIOLOGY 2003. THESSALONIKI, GREECE, SEPT. 21 - 24, 2003; [COMPUTERS IN CARDIOLOGY], NEW YORK, NY : IEEE, US, vol. VOL. 30, 21 September 2003 (2003-09-21), pages 247-250, XP010698886, DOI: 10.1109/CIC.2003.1291137 ISBN: 978-0-7803-8170-4**

**Description**

FIELD OF THE INVENTION

The invention relates to an apparatus, a system and a method for measuring physical properties inside a vessel.

BACKGROUND OF THE INVENTION

Functional impact of a stenosis of a blood vessel is typically quantified by fractional flow reserve (FFR) or instantaneous wave-free ratio (iFR) value. The FFR value is calculated as a ratio of pressures measured distal and proximal to the stenosis. For the calculation of the iFR value a specific wave-free period is isolated during the diastole phase of the heartbeat cycle, and the ratio of pressures distal and proximal to the stenosis is ascertained over this period. During this wave-free period, the competing forces (waves) that affect the blood flow are quiescent, meaning that pressure and flow are linearly related as compared to the rest of the cardiac cycle. According to the FFR or iFR value the severity of the stenosis is established, specifically whether the stenosis limits blood flow within the vessel to an extent which necessitates intervention.

US 2015/105673 A1 discloses a method for calculating fractional flow reserve comprising the steps of: advancing a pressure sensing guidewire through a blood vessel to a first position distal of an intravascular occlusion, determining a distal pressure at the first position of the pressure sensing guidewire, proximally shifting the pressure sensing guidewire to a second position proximal of the occlusion, determining a proximal pressure at the second position of the pressure sensing guidewire, measuring an aortic pressure, and calculating a pressure drift as a difference between the aortic pressure and the proximal pressure and calculating a drift-compensated FFR, wherein the drift-compensated FFR corresponds to the sum of the distal pressure and the pressure drift, divided by the aortic pressure.

WO 2016/030491 A1 discloses a method of tracking cardiac cycle events and evaluating a cardiac system, the method comprising determining a plurality of distal pressure to proximal pressure Pd/Pa ratios, determining a minimum Pd/Pa ratio from the plurality of Pd/Pa ratios; and displaying the minimum Pd/Pa ratio.

US 2016/106373 A1 discloses methods for calculating a corrected Fractional Flow Reserve. Methods include delivering a pressure sensing device including a pressure sensor to a location in an artery having a stenosis, positioning the pressure sensor distal to the stenosis, measuring the distal pressure, measuring the proximal pressure, and calculating a corrected Fractional Flow Reserve using the measured proximal and distal pressures and applying a correction factor or correction equation. The correction factor or correction equation corrects for changes in the measured distal pressure caused by a presence of the pressure sensing device. A data set of correction factors or correction equations may be stored in a memory component of the system. The corrected Fractional Flow reserve may approximate the Fractional Flow Reserve that would be obtained if a different sized device was used to measure the distal pressure, such as a pressure sensing guidewire having an about 0.356 mm (0.014 inch) outer diameter.

US 2016/073972 A1 relates to methods for evaluating a vessel of a patient. A disclosed method includes outputting, to a touch-sensitive display of a bedside controller, a screen display including: a visual representation of a first pressure ratio of pressure measurements obtained by first and second instruments positioned within a vessel while the second instrument is moved from a distal position to a proximal position relative a stenosis and the first instrument remains stationary; and a first proximal pressure waveform and a first distal pressure waveform; receiving, through the touch-sensitive display of the bedside controller, a user touch input on the first proximal pressure waveform and/or the first distal pressure waveform identifying a time at which pressure measurements were obtained; and modifying the screen display, in response to the user touch input, to further include a visual representation of the obtained pressure measurements corresponding to the identified time.

SUMMARY OF THE INVENTION

It is an object of the invention to provide an apparatus, a system and a method for feedback on quality of physical property measurement in a blood vessel.

According to the invention, this object is realized by an apparatus for measurement of a property of a pulsatile blood motion within a vessel of a living being, the apparatus configured to:

   receive a first temporal measurement signal from a first sensor and a second temporal measurement signal from a second sensor, the first and second temporal measurement signals indicative of the property of the pulsatile blood motion within the vessel;
   normalize the first and second temporal measurement signals when the first and second sensors are in a predetermined relative spatial position within the vessel;
   ascertain a figure of merit based on a shape deviation between the temporal measurement signals after normalization;

and
output to a user interface the figure of merit.

The figure of merit, ascertained based on anomalies regarding the shapes of the temporal measurement signals, is an indication of the trustworthiness of the measurements.

In an embodiment of the apparatus, the duration of the temporal measurement signals is a period of the pulsatile blood motion within the vessel. The figure of merit determined for a single cardiac cycle takes advantage of the fact that the measurement signals show repetitive patterns, and improvement or deterioration of the figure of merit can be monitored for subsequent cardiac cycles.

In an embodiment, the first and second temporal measurement signals are pressure measurements. In a further embodiment the first and second temporal measurement signals are blood flow velocity measurements. Pressure measurements and blood flow velocity measurements follow the pulsatile nature of the blood motion within the vessel of the living being, and they provide crucial information on the condition of the blood vessel.

In an embodiment, the apparatus comprises the user interface configured to display the figure of merit. Arrangement of medical instrumentation in an operation room benefits from compact apparatus with integrated user interface.

In an embodiment, the figure of merit is displayed as a numerical value. Interpretation of numerical values from a numerical range creates awareness of the user on how far the actual circumstances are situated from the two extrema of the numerical range, and consequently on how far the actual figure of merit is from the most optimal situation.

In an embodiment, the figure of merit is displayed as a color. Color coding of acceptable values of the figure of merit has the benefit that the user can assess quickly whether the measurement signals are of at least the minimal necessary quality, without being tempted to further improve the figure of merit toward the most optimal situation.

In an embodiment, the apparatus is further configured to:

ascertain a cause of the shape deviation between the temporal measurement signals after normalization, when the figure of merit is below a predetermined threshold value;
output to a user interface the cause of the shape deviation.

Causes of various shape deviations between the temporal measurement signals may be determined empirically in clinical practice. Algorithms may detect various shape deviations, such as slope differences, discrepancy of numbers of extrema in the measurement signals for a finite duration (e.g. for a period of a cardiac cycle), spurious peaks in the measurement signals. The various shape deviations are associated to the empirically determined causes. An experienced user of the apparatus can take corrective actions to improve the figure of merit by knowing the cause of the shape deviation.

In an embodiment, the apparatus is further configured to:

ascertain an action of remedy to eliminate the cause of the shape deviation between the temporal measurement signals;
output to a user interface the action of remedy.

The action of remedy to eliminate the cause of the shape deviation between the temporal measurement signals may be determined empirically in clinical practice, since the action of remedy is directly linked to the cause of the shape deviation. A less experienced user of the apparatus is able to proceed faster in the clinical workflow upon a suggested action of remedy of the shape deviation, which presents benefit for the patient and for the medical institution.

In an embodiment, the apparatus is configured to:

ascertain an FFR or an iFR value; and
output the FFR or iFR value to a user interface.

FFR and iFR are the most used techniques for assessment of the functional impact of a stenosis of the blood vessel. A user informed on the figure of merit while assessing FFR or iFR, can evaluate the trustworthiness of the assessment based on the figure of merit, and may contemplate to repeat the assessment or to associate treatment options to the condition of the vessel.

In a different embodiment, the apparatus comprises the user interface configured to display the figure of merit and to display the FFR or iFR value selectively, when the figure of merit is above a predetermined threshold value. A less experienced user is prevented to assess the condition of the vessel based on an FFR or iFR value in case of a poor figure of merit.

In an embodiment, the apparatus is further configured to:
emit a warning signal when the figure of merit is below a predetermined threshold value. The attention of the user is attracted by the warning signal quickly to the fact that a poor figure of merit compromises the trustworthiness of the measurement signals. The warning signal may be an auditive signal or a visual warning signal on a user interface.

In another aspect of the invention, a system comprises a medical instrument and the apparatus according to the invention, wherein the medical instrument comprises the first and second sensors, and wherein a relative position between the first and second sensors is adjustable. The medical instrument may be a catheter with telescopically extendable distal portion, or a guiding catheter-guidewire assembly. The adjustable relative position between the first and second sensors plays a role in normalization of the measurement signals for a predetermined relative position of the sensors, usually in close proximity to each other, followed by a functional measurement by adjusting the distance between the two sensors such that one of the sensors is proximal and the other one is distal to the segment of the vessel of which the condition is assessed.

In a different aspect of the invention, a method of assessing the quality of the measurement of the property of the pulsatile blood motion within the vessel of the living being is presented, comprising:

receiving a first temporal measurement signal from a first sensor and a second temporal measurement signal from a second sensor, the first and second temporal measurement signals indicative of the property of the pulsatile blood motion within the vessel;

normalizing the first and second temporal measurement signals when the first and second sensors are in a predetermined relative spatial position within the vessel;

ascertaining a figure of merit based on a shape deviation between the temporal measurement signals after normalization; and

outputting to a user interface the figure of merit.

The method enables assessment of the quality of the measurements, and supports the user to decide whether to proceed with diagnostic functional measurements, which form the bases of the selection of specific treatment options (e.g. angioplasty, stent deployment) based on the condition of the vessel segment.

Additional aspects and advantages of the invention will become more apparent from the following detailed description, which may be best understood with reference to and in conjunction with the accompanying drawings.

BRIEF DESCRIPTION OF THE DRAWINGS

In the drawings:

Fig. 1 shows schematically and exemplarily an embodiment of a system according to the invention.

Fig. 2 shows schematically and exemplarily an embodiment of a medical instrument used for measuring physical properties within a blood vessel.

Fig. 3 shows exemplary a temporal measurement signal indicative of pulsatile blood pressure, provided by the first sensor integrated into the medical instrument.

Fig. 4 shows exemplary a temporal measurement signal indicative of pulsatile blood pressure, provided by the second sensor integrated into the medical instrument.

Fig. 5 shows exemplarily the first and second temporal pressure measurement signals normalized.

Fig. 6 shows exemplarily the user interface according to the invention.

Fig. 7 shows schematically a method of assessing the quality of the measurement of a property of a pulsatile blood motion within a vessel of a patient, according to the invention.

DETAILED DESCRIPTION OF EMBODIMENTS

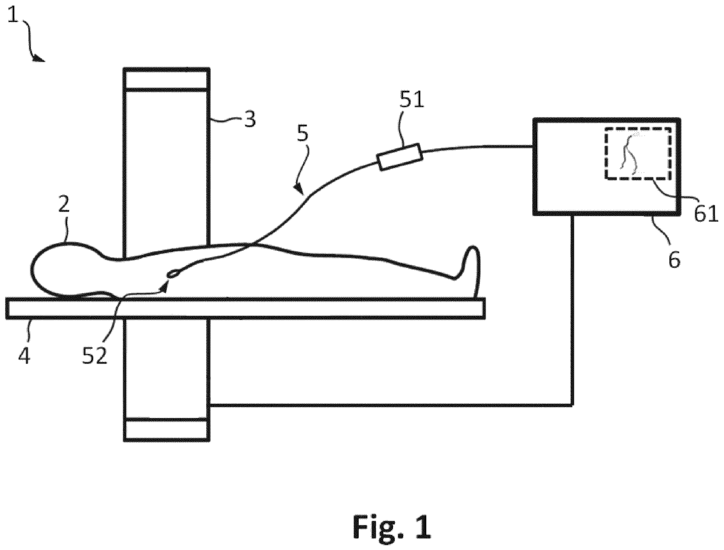

Fig. 1 shows schematically and exemplarily an embodiment of a system 1 according to the invention, used for measurement of a property of a pulsatile blood motion within a vessel of a living being 2. The system comprises a medical instrument 5 extending from the handgrip 51 to a distal portion 52, which is introduced into the vasculature of a patient 2 resting on a support means 4. Sensors integrated into the distal portion of the medical instrument 5 enable measurements of physical properties of blood within the vessel. The measurement signals are transmitted to an apparatus 6 for further signal conditioning and/or deriving additional blood flow related characteristics. The apparatus 6 is configured to render representations of the measurement signals and/or blood flow related characteristics on a display of a user interface 61.

The system further comprises an imaging unit 3 for acquiring measurement information indicative of the morphology of the vessel. The imaging unit is one of a radiological, a magnetic resonance, and an ultrasound imaging unit, further providing measurement information on the position of at least a distal portion of the medical instrument 5 with respect to the morphology of the target vessel. A representation of the morphology of the vessel together with either the position of the distal portion 52 or that of a segment of the medical instrument 5 present in the field of view of the imaging unit 3 can be rendered on the display of the user interface 61. Contrast agents may be injected into the blood stream for enhancing the visibility of the vessel in the morphological representation.

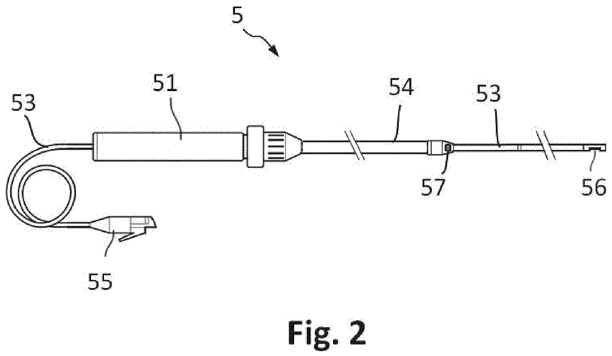

A guiding catheter-guidewire assembly shown in Fig. 2 is used as medical instrument 5 for assessment of the condition of the vessel. The guidewire 53, is inserted into the vasculature of the patient 2 through a lumen of the guiding catheter 54 formed by a tubular elongate body. A sensor 57 is integrated into the distal portion of the guiding catheter 54 for providing measurement of a physical property of the pulsatile blood motion within the vessel. In an alternative embodiment, the pressure at the position of the distal end of the guiding catheter in the blood vessel is measured outside the body with a transducer connected to the guiding catheter and being accessed by the blood through the lumen of the guiding catheter. The distal portion of the guidewire 53 comprises another sensor 56. The sensors 56 and 57 are suitable for providing at least one of a blood pressure and a blood flow velocity measurements. Typical examples for guidewires are the Verrata® pressurewire and the FloWire®Doppler guidewire, enabling pressure and blood flow velocity measurements respectively, both being manufactured by Volcano Corporation. The guidewire may comprise multiple sensors distributed spatially along the distal portion. The sensors 56 and 57 may employ one of the various techniques for blood pressure and/or blood flow measurements. Typical pressure sensors use change of piezoresitive property upon pressure, capacitive readout of a suspended membrane over a substrate or fluidic transduction of a deflection of a diaphragm to a mechanical or an electrical signal. In an embodiment, both sensors enable simultaneous measurement of multiple physical properties, for example a Capacitive Micromachined Ultrasonic Transducer (CMUT) provides blood pressure measurement by capacitive readout of the suspended membrane, while the same CMUT sensor is used for providing blood flow velocity measurement by using ultrasound Doppler technique.

The most frequently used assessment of the functional impact of a stenosis within a blood vessel is based on an FFR or iFR value, ascertained from a distal pressure Pd measured by the sensor 56 on the guidewire 53 at one side of the stenosis, and a proximal pressure Pa measured by the sensor 57 on the guiding catheter 54 at the other side of the stenosis. The medical instrument 5 is configured such that the guidewire 53 is movable within the guiding catheter 54, hence the position of the sensor 56 is adjustable with respect to the position of the sensor 57. Sensors 56 and 57 should ideally measure the same pressure at the same location within the blood vessel, however this rarely occurs due to inter-sensor variability. A reliable assessment of the functional impact of the stenosis requires a normalization of the pressure measurements provided by the sensors 56 and 57 at a relative position of close proximity to each other, in order to compensate for the inter-sensor variability. Normalization of the measurement signals is performed for the guiding catheter-guidewire assembly in a predetermined configuration, where for instance the sensor 56 on the guidewire 53 is just about distal to the distal end of the guiding catheter 54. Normalization can also compensate for latency differences (in the time domain). In an alternative embodiment, the pressures can be measured via different measurement paths along the vessel, and the latency difference of the two pressure measurement signals is compensated by normalization.

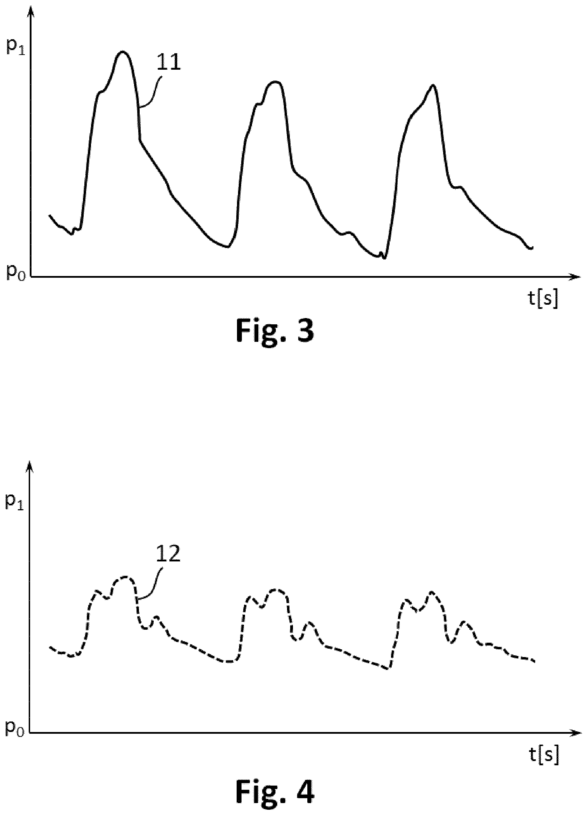

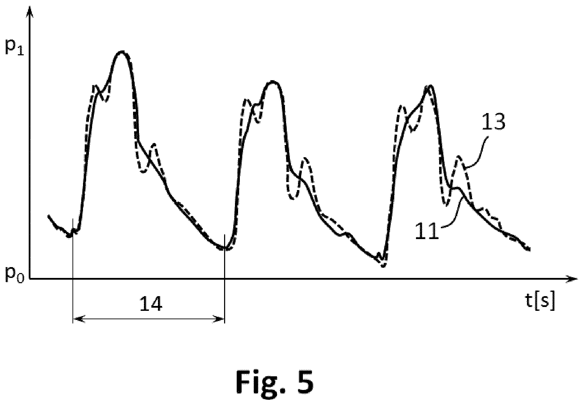

Figs. 3 to 5 show an exemplarily case in a graphical representation. The temporal measurement signal 11 of the pulsatile blood pressure shown in Fig. 3 is provided by the sensor 56 on the guidewire 53. This differs in magnitude and by an offset (in pressure and/or in time domain) from the temporal measurement signal 12 of the pulsatile blood pressure shown in Fig. 4, provided by the sensor 57 on the guiding catheter 54. The apparatus 6 is adapted to normalize the temporal measurement signals 11 and 12, such that the offset and the magnitude differences are minimized. After normalization the signals are expected to overlap and the ratio of Pd/Pa to approach 1, in other words the FFR or iFR value of two pressures measured in two locations proximate to each other within the blood vessel to result in a value 1. In the exemplary embodiment the measurement signal 12 is stretched and compensated for offset in a manner that the resulting proximal pressure Pa measurement signal 13 overlaps significantly with the distal pressure Pd measurement signal 11. The best overlap is optimized based on cost function. Due to various circumstances however, the temporal measurement signals 11 and 13 have portions where they do not overlap in clinical practice, as shown in Fig. 5. Although the difference of the mean pressures calculated for the two pressure measurement signals is significantly small after the normalization step, certain anomaly is evident from the partial divergence of the two signals in Fig. 5. A continuation of the clinical workflow to extend the guidewire 53 beyond a vessel segment comprising a stenosis to assess the FFR value at this point in time is inappropriate, since the assessment will be based on a proximal pressure signal 13 comprising anomalies, hence resulting in an incorrect FFR value.

The apparatus 6 is configured to detect the anomalies causing the partial discrepancies of the pressure measurement signals 11 and 13. Detection of the anomalies is based on one of the following techniques: ascertaining the average absolute difference between samples of the two signals taken at the same time, cross-correlation between the two signals, comparison of the slopes of the two signals, ascertaining the numbers of local extrema (maxima and minima) in a period 14 of the cyclical heartbeat. The apparatus 6 is further configured to ascertain a figure of merit based on a combination of the type of anomaly and the magnitude of the discrepancy between the measurement signals caused by the anomaly. In the exemplary embodiment the figure of merit C is ascertained with:

$$C = 1 - \frac{\Delta}{(\Delta + \alpha)} \qquad\qquad (Eq.\,1)$$

where $\alpha$ is a constant to tune the function, typically in the range 0.1 - 10;
$\Delta = \sum_n (Pd_i - Pa_i)^2 / n$; and
n is the number of pressure measurement values used for normalization.

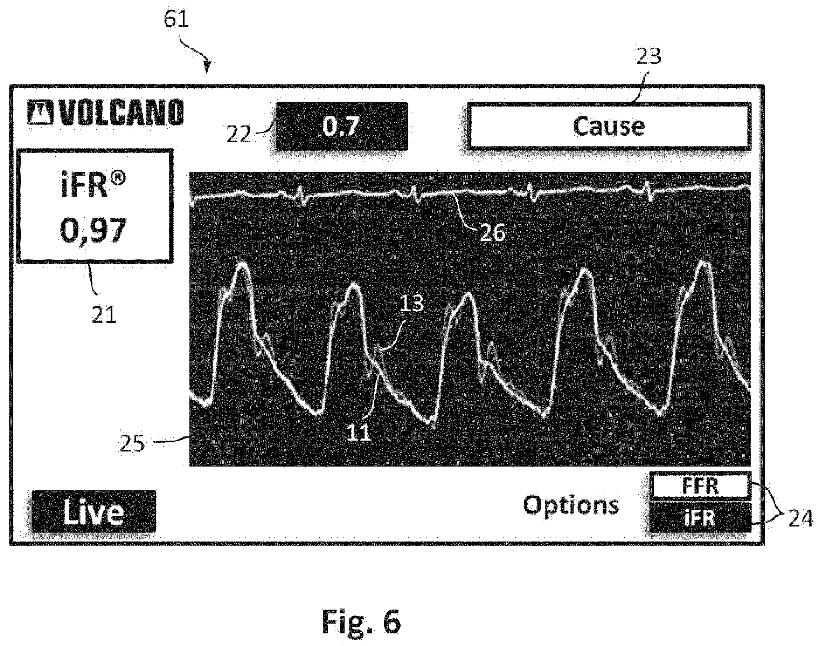

The figure of merit 22 is displayed on the user interface 61, exemplarily illustrated in Fig. 6. In the exemplary embodiment the figure of merit takes values in the interval of 0 to 1, however, in alternative embodiments an interval is selected by the user (e.g. 0 to 100, -1 to 1, etc.) and the values obtained with *Eq.* 1 are associated to numerical values in the selected interval according to a linear or logarithmic scale. In an embodiment, the tab assigned to the figure of merit 22 is displayed in color, wherein a perfect overlap of the two signals 11 and 13 is indicated with green and a poor match of the signals is indicated with red. Other variations of colors are possible. Particular ranges in the numerical value interval of the figure of merit may be assigned specific colors according to a colorbar.

The user interface 61 may further comprise a tab indicating the cause 23 of the anomaly of the pressure measurement signals. The anomaly in the exemplary embodiment illustrated in Figs. 3-6 is caused by the guiding catheter 54 comprising the proximal pressure measurement sensor 57 entering the ostium of the coronary artery. In an embodiment, the user interface may comprise a tab (not shown) for presenting suggestion to the user on remedies for correcting the anomaly. Alternatively, the action to remedy the cause is displayed together with the cause of the anomaly. A repositioning of the guiding catheter is the appropriate remedy in the exemplary embodiment.

Other frequent anomalies and related causes are, but not limited to: the Pa measurement signal presents shallow slopes and fewer extrema than the Pd measurement signal, which is caused by overdamping of the Pa signal; the Pa measurement signal presents spurious peaks, which is caused by the guiding catheter tapping against the ostium of the artery in which the Pd signal is measured; the Pa measurement signal presents more higher frequencies than usual, which is caused by underdamping of the measurement signal. The causes of various anomalies are empirically determined by using statistical information from clinical practice.

The user interface 61 further comprises tabs 24 for selecting FFR or iFR assessment, and a window 21 in which the ascertained FFR or iFR value is presented to the user. The two pressure measurement signals Pd and Pa are shown to the user in a measurement window 25, wherein optionally an electrocardiogram or an electrogram 26 is further presented.

In an embodiment, the apparatus 6 is further configured to detect anomalies of the pressure measurement signals also during the assessment of the functional impact of the stenosis on the blood flow in the vessel by FFR or iFR, wherein the sensor 56 on the guidewire 53 is advanced distal to the stenosis and the sensor 57 on the guiding catheter is kept proximal to the stenosis of the vessel. Upon detection of anomalies with respect to the shape of the pressure measurement signals by the apparatus, the user is presented the figure of merit 22 on the user interface 61, which reflects the trustworthiness of the FFR or iFR value. Frequent anomalies during FFR or iFR measurements are, but not limited to: fewer extrema in either of the pressure measurement signals than in the other one, spurious peaks in the measurement signals, sudden discrepancy between the slopes of the measurement signals. Similar to the normalization step, the cause 23 of the anomaly during FFR or iFR assessment is presented in the tab of the user interface 61, optionally together with the suggestion of remedy for correction of the anomaly.

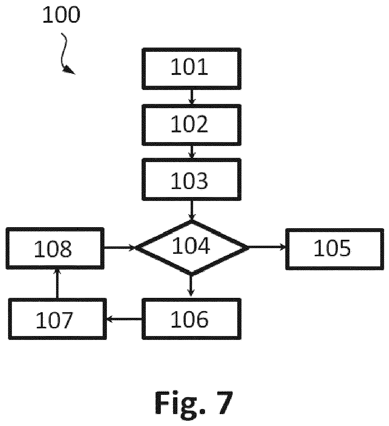

In Fig. 7 a method 100 of assessing the quality of the measurement of a property of the pulsatile blood motion within the vessel of a patient is presented schematically. In step 101 the apparatus receives a first temporal measurement signal 11 from a first sensor 56 and a second temporal measurement signal 12 from a second sensor 57, the first and second temporal measurement signals 11,12 being indicative of the property of the pulsatile blood motion within the vessel. This can be achieved by introducing the distal portion of the medical instrument into the vessel of the patient, the medical instrument 5 comprising the first sensor 56 and the second sensor 57 with adjustable relative spatial positions with respect to each other. The medical instrument may be a guiding catheter-guidewire assembly 53,54 or a catheter with telescopically extendable distal portion. The sensor 56 on the guidewire 53 is brought in predetermined relative spatial position within the vessel with respect to the sensor 57 on the guiding catheter 54, for instance just about distal to the distal end of the guiding catheter 54. In step 102 the user initializes normalization of the first and second temporal measurement signals 11,12 when the first and second sensors 56,57 are in the predetermined relative spatial position within the vessel. In the exemplary embodiment the pressure measurement signals 11 and 12 are normalized based on the mean pressures values, resulting in pressure measurement signals 11 and 13, graphically illustrated in Fig. 5. In step 103 the normalized signals 11, 13 are compared, and a figure of merit 22 is ascertained based on the shape deviation between the temporal measurement signals 11, 13 after normalization. In step 104 the figure of merit 22 is output to the user interface 61 and an evaluation of the normalized measurement signals is performed. In case that the figure of merit indicates no anomalies that can detrimentally influence assessment of the functional impact of a stenosis, the user may proceed to FFR or iFR assessment in step 105, by extending the guidewire distal to the stenosis or distal to the segment of blood vessel suspected to comprise a lesion. In the other case, when the figure of merit is poor and the anomalies can detrimentally influence the FFR or iFR assessment, the user is informed in step 106 that the FFR and iFR measurements are not trustworthy. In an embodiment, the user is presented through the user interface 61 a cause of the anomaly in step 107, and optionally in step 108 the user is informed on the appropriate action to remedy the anomalies.

Although pulsatile blood pressure measurement has been used for the elucidation of the invention, similar methodology can be applied when the sensors 56 and 57 are used for blood flow velocity measurements of the pulsatile blood motion.

Other variations to the disclosed embodiments can be understood and effected by those skilled in the art in practicing the claimed invention, from a study of the drawings, the disclosure, and the appended claims.

A single unit or device may fulfill the functions of several items recited in the claims. The mere fact that certain measures are recited in mutually different dependent claims does not indicate that a combination of these measures cannot be used to advantage.

In the claims, the word "comprising" does not exclude other elements or steps, and the indefinite article "a" or "an" does not exclude a plurality.

Any reference signs in the claims should not be construed as limiting the scope.

**Claims**

1. An apparatus (6) for measurement of a property of a pulsatile blood motion within a vessel of a living being (2), the apparatus configured to:

   receive a first temporal measurement signal (11) from a first sensor (56) and a second temporal measurement signal (12) from a second sensor (57), the first and second temporal measurement signals (11,12) indicative of the property of the pulsatile blood motion within the vessel;
   normalize the first and second temporal measurement signals (11,12) when the first and second sensors (56,57) are in a predetermined relative spatial position within the vessel;
   **characterized in that** the apparatus is further configured to:

      ascertain a figure of merit (22) based on a shape deviation between the temporal measurement signals (11,13) after normalization, the figure of merit (22) being indicative of trustworthiness of the temporal measurement signals (11,13); and
      output to a user interface (61) the figure of merit (22).

2. The apparatus (6) according to claim 1, wherein a duration of the temporal measurement signals (11,12) is a period (14) of the pulsatile blood motion within the vessel.

3. The apparatus (6) according to claim 1, wherein the first and second temporal measurement signals (11,12) are pressure measurements.

4. The apparatus (6) according to claim 1, wherein the first and second temporal measurement signals (11,12) are blood flow velocity measurements.

5. The apparatus (6) according to claim 1, further comprising the user interface (61) configured to display the figure of merit (22).

6. The apparatus (6) according to claim 1, wherein the figure of merit (22) is displayed as a numerical value.

7. The apparatus (6) according to claim 1, wherein the figure of merit (22) is displayed as a color.

8. The apparatus (6) according to claim 1, further configured to:

   ascertain a cause (23) of the shape deviation between the temporal measurement signals (11,13) after normalization, when the figure of merit (22) is below a predetermined threshold value;
   output to a user interface (61) the cause (23) of the shape deviation.

9. The apparatus (6) according to claim 8, further configured to:

ascertain an action of remedy to eliminate the cause of the shape deviation between the temporal measurement signals (11,13);
output to a user interface (61) the action of remedy.

10. The apparatus (6) according to claim 1, further configured to:

ascertain a fractional flow reserve FFR or an instantaneous wave-free ratio iFR value (21) ; and
output the FFR or iFR value (21) to a user interface (61).

11. The apparatus (6) according to claim 1, further comprising the user interface (61) configured to display the figure of merit (22) and to display the FFR or iFR value (21) selectively, when the figure of merit (22) is above a predetermined threshold value.

12. The apparatus (6) according to claim 1, further configured to:
emit a warning signal when the figure of merit (22) is below a predetermined threshold value.

13. A system (1) comprising the apparatus (6) according to claim 1 and a medical instrument (5), wherein the medical instrument (5) comprises the first and second sensors (56,57), and wherein a relative position between the first and second sensors (56,57) is adjustable.

14. The system (1) according to claim 13, further comprising the user interface (61), wherein the medical instrument (5) is a guiding catheter-guidewire assembly (53,54), and wherein the guidewire (53) comprises the first sensor (56) and the guiding catheter (54) comprises the second sensor (57).

15. A method (100) of assessing a quality of a measurement of a property of a pulsatile blood motion within a vessel of a living being (2), comprising:

receiving (101) a first temporal measurement signal (11) from a first sensor (56) and a second temporal measurement signal (12) from a second sensor (57), the first and second temporal measurement signals (11,12) indicative of the property of the pulsatile blood motion within the vessel;
normalizing (102) the first and second temporal measurement signals (11,12) when the first and second sensors (56,57) are in a predetermined relative spatial position within the vessel;
ascertaining (103) a figure of merit (22) based on a shape deviation between the temporal measurement signals (11,13) after normalization, the figure of merit (22) being indicative of trustworthiness of the temporal measurement signals (11,13); and
outputting (104) to a user interface (61) the figure of merit (22).

**Patentansprüche**

1. Vorrichtung (6) zum Messen einer Eigenschaft einer pulsierenden Blutbewegung innerhalb eines Gefäßes eines Lebewesens (2), wobei die Vorrichtung konfiguriert ist zum:

Empfangen eines ersten zeitlichen Messsignals (11) von einem ersten Sensor (56) und eines zweiten zeitlichen Messsignals (12) von einem zweiten Sensor (57), wobei die ersten und zweiten zeitlichen Messsignale (11,12) die Eigenschaft der pulsierenden Blutbewegung innerhalb des Gefäßes anzeigen;
das erste und das zweite zeitliche Messsignal (11,12) zu normieren, wenn sich der erste und der zweite Sensor (56,57) in einer vorbestimmten relativen räumlichen Position innerhalb des Gefäßes befinden;
**dadurch gekennzeichnet, dass** der Apparat außerdem konfiguriert ist zum:

Bestimmen einer Gütezahl (22) basierend auf einer Formabweichung zwischen den zeitlichen Messsignalen (11, 13) nach Normalisierung, wobei die Gütezahl (22) die Vertrauenswürdigkeit der zeitlichen Messsignale (11,13) anzeigt; und
Ausgabe der Gütezahl (22) an eine Benutzerschnittstelle (61) .

2. Vorrichtung (6) nach Anspruch 1, wobei eine Dauer der zeitlichen Messsignale (11,12) eine Periode (14) der pulsierenden Blutbewegung innerhalb des Gefäßes ist.

3. Vorrichtung (6) nach Anspruch 1, wobei das erste und das zweite zeitliche Messsignal (11,12) Druckmessungen sind.

4. Vorrichtung (6) nach Anspruch 1, wobei das erste und das zweite zeitliche Messsignal (11,12) Messungen der Blutflussgeschwindigkeit sind.

5. Vorrichtung (6) nach Anspruch 1, ferner umfassend die Benutzerschnittstelle (61), die so konfiguriert ist, dass sie die Gütezahl (22) anzeigt.

6. Vorrichtung (6) nach Anspruch 1, wobei die Gütezahl (22) als numerischer Wert angezeigt wird.

7. Vorrichtung (6) nach Anspruch 1, wobei die Gütezahl (22) als Farbe angezeigt ist.

8. Vorrichtung (6) nach Anspruch 1, ferner konfiguriert zum:

   Ermitteln einer Ursache (23) der Formabweichung zwischen den zeitlichen Messsignalen (11,13) nach Normierung,
   wenn die Gütezahl (22) unter einem vorgegebenen Schwellenwert liegt;
   Ausgabe der Ursache (23) der Formabweichung an eine Benutzerschnittstelle (61).

9. Vorrichtung (6) nach Anspruch 8, ferner konfiguriert zum:

   Ermitteln einer Abhilfemaßnahme zur Beseitigung der Ursache der Formabweichung zwischen den zeitlichen Messsignalen (11,13);
   Ausgabe der Abhilfemaßnahme an eine Benutzerschnittstelle (61).

10. Vorrichtung (6) nach Anspruch 1, ferner konfiguriert zum:

    Ermitteln einer Teilflussreserve FFR oder eines momentanen wellenfreien Verhältnis-iFR-Werts (21); und
    Ausgeben des FFR- oder iFR-Werts (21) an eine Benutzerschnittstelle (61).

11. Vorrichtung (6) nach Anspruch 1, ferner umfassend die Benutzerschnittstelle (61), die konfiguriert ist, um die Gütezahl (22) anzuzeigen und den FFR- oder iFR-Wert (21) selektiv anzuzeigen, wenn die Gütezahl (22) über einem vorbestimmten Schwellenwert ist.

12. Vorrichtung (6) nach Anspruch 1, ferner konfiguriert zum:
    Abgeben eines Warnsignals, wenn die Gütezahl (22) unter einem vorgegebenen Schwellwert liegt.

13. System (1), das die Vorrichtung (6) nach Anspruch 1 und ein medizinisches Instrument (5) umfasst, wobei das medizinische Instrument (5) den ersten und den zweiten Sensor (56,57) umfasst und wobei eine relative Position zwischen dem ersten und zweite Sensoren (56,57) einstellbar ist.

14. System (1) nach Anspruch 13, ferner umfassend die Benutzerschnittstelle (61), wobei das medizinische Instrument (5) eine Führungskatheter-Führungsdraht-Anordnung (53,54) ist,
    und wobei der Führungsdraht (53) den ersten Sensor (56) umfasst und der Führungskatheter (54) den zweiten Sensor (57) umfasst.

15. Verfahren (100) zum Bewerten einer Qualität einer Messung einer Eigenschaft einer pulsierenden Blutbewegung in einem Gefäß eines Lebewesens (2), umfassend:

    Empfangen (101) eines ersten zeitlichen Messsignals (11) von einem ersten Sensor (56) und eines zweiten zeitlichen Messsignals (12) von einem zweiten Sensor (57), wobei die ersten und zweiten zeitlichen Messsignale (11,12) die Eigenschaft der pulsierenden Blutbewegung innerhalb des Gefäßes anzeigen;
    Normieren (102) des ersten und des zweiten zeitlichen Messsignals (11,12), wenn sich der erste und der zweite Sensor (56,57) in einer vorbestimmten relativen räumlichen Position innerhalb des Gefäßes befinden;
    Bestimmen (103) einer Gütezahl (22) basierend auf einer Formabweichung zwischen den zeitlichen Messsignalen (11,13) nach Normalisierung, wobei die Gütezahl (22) eine Anzeige der Vertrauenswürdigkeit der zeitlichen Messsignale (11,13) ist; und Ausgeben (104) der Gütezahl (22) an eine Benutzerschnittstelle (61).

**Revendications**

1. Un appareil (6) pour mesurer la propriété d'un mouvement sanguin pulsatile à l'intérieur d'un vaisseau sanguin d'un être vivant (2), l'appareil est configuré pour :

   recevoir le premier signal de mesure temporelle (11) à partir d'un premier capteur (56) et d'un second signal de mesure temporelle (12) à partir d'un second capteur (57), les premiers et seconds signaux de mesure temporelle (11,12) indiquent la propriété d'un mouvement sanguin pulsatile à l'intérieur du vaisseau ; normaliser les signaux des premières et secondes mesures temporelles (11,12) lorsque les premiers et seconds capteurs (56,57) se trouvent dans une position spatiale relative prédéterminée à l'intérieur du vaisseau sanguin ; **caractérisé par le fait que** l'appareil est également configuré pour :

   vérifier une figure de proue (22) fondée sur une forme de dérivation entre les signaux de mesure temporale (11,13) après la normalisation, la figure de proue (22) étant indicative de la fiabilité des signaux de mesure temporelle (11,13) ; et sortir vers une interface utilisateur (61) la figure de proue (22).

2. L'appareil (6) selon la revendication 1, où la durée des signaux des mesures temporelles (11,12) représente la période (14) du mouvement sanguin pulsatile à l'intérieur du vaisseau sanguin.

3. L'appareil (6) selon la revendication 1, où les premiers et seconds signaux de mesure temporelle (11,12) sont des mesures de pression.

4. L'appareil (6) selon la revendication 1, où les premiers et seconds signaux de mesure temporelle (11,12) représentent des mesures de la vitesse du flux sanguin.

5. L'appareil (6) selon la revendication 1, comprend également l'interface utilisateur (61) configurée pour afficher la figure de proue (22).

6. L'appareil (6) selon la revendication 1, où la figure de proue (22) est affichée en tant que valeur numérique.

7. L'appareil (6) selon la revendication 1, où la figure de proue (22) est affichée en tant que couleur.

8. L'appareil (6) selon la revendication 1 est également configuré pour :

   déterminer une cause (23) de l'écart de forme entre les signaux de mesure temporelle (11,13) après la normalisation, lorsque la figure de proue (22) se trouve en dessous d'une valeur d'un seuil prédéterminé ; une sortie vers une interface utilisateur (61) la cause (23) de l'écart de forme.

9. L'appareil (6) selon la revendication 8 est également configuré pour :

   vérifier l'action de recours pour éliminer la cause d'un écart de forme entre les signaux de mesures temporelles (11,13) ; sortir vers l'interface utilisateur (61) l'action en recours.

10. L'appareil (6) selon la revendication 1 est également configuré pour :

    vérifier une réserve de débit fractionnaire FFR ou une valeur d'un rapport instantané sans ondes (21) ; et sortir la valeur FFR ou iFR (21) vers une interface utilisateur (61).

11. L'appareil (6) selon la revendication 1, comprend également l'interface utilisateur (61) configurée pour afficher la figure de proue (22) et pour afficher les valeurs FFR ou iFR (21) de manière sélective, lorsque la figure de proue (22) se trouve au-dessus d'une valeur d'un seuil prédéterminée.

12. L'appareil (6) selon la revendication 1 est également configuré pour :

émettre un signal d'avertissement lorsque la figure de proue (22) se trouve en dessous d'un seuil de valeur prédéterminé.

13. Un système (1) comprend l'appareil (6) selon la revendication 1 et un instrument médical (5), où l'instrument médical (5) comprend les premiers et seconds capteurs (56,57), et où une position relative entre les premiers et seconds capteurs (56,57) est ajustable.

14. Le système (1) selon la revendication 13 comprend également l'interface utilisateur (61), où l'instrument médical (5) est un ensemble cathéter de guidage - guide-fil (53,54), et où le guidage - guide-fil (53) comprend le premier capteur (56) et le cathéter de guidage (54) comprend un second capteur (57).

15. Une méthode (100) pour évaluer la qualité d'une mesure d'une propriété d'un mouvement sanguin pulsatile à l'intérieur d'un vaisseau sanguin d'un être vivant (2), comprend:

la réception (101) d'un premier signal de mesure temporelle (11) à partir d'un premier capteur (56) et d'un second signal de mesure temporelle (12) à partir d'un second capteur (57), les premiers et seconds signaux de mesure temporelle (11,12) indiquent la propriété du mouvement sanguin pulsatile à l'intérieur du vaisseau sanguin;

la normalisation (102) des premiers et seconds signaux de mesure temporelle (11,12) lorsque les premiers et seconds capteurs (56,57) se trouvent dans une position spatiale relative prédéterminée à l'intérieur du vaisseau sanguin;

vérifier (103) une figure de proue (22) fondée sur un écart de forme entre les signaux des mesures temporelles (11,13) après la normalisation, la figure de proue (22) étant indicative de la fiabilité des signaux de mesures temporelles (11,13); et

la sortit (104) d'une interface utilisateur (61) la figure de proue (22).

**Fig. 1**

EP 3 484 346 B1

Fig. 2

**Fig. 3**

**Fig. 4**

**Fig. 5**

Fig. 6

Fig. 7

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2015105673 A1
- WO 2016030491 A1
- US 2016106373 A1
- US 2016073972 A1